# EUROPEAN PATENT APPLICATION

(11) **EP 4 810 514 A1**
(43) Date of publication of application: **23.09.2026**
(21) Application number: 24891806.2
(22) Date of filing: 15.11.2024
(51) Int. Cl.: C07K 16/18, A61P 35/00, A61K 38/00, A61K 39/00

(54) **SKP2 FRAGMENT, VARIANT THEREOF, AND USE THEREOF**

(30) Priority: 15.11.2023 KR 20230158319
(71) Applicant: Genexine, Inc., Seoul 07789 (KR)
(72) Inventor: YONG, Yeryoung, Seoul 06544 (KR); LEE, Seunghyun, Seoul 02720 (KR); CHOI, Jaehyun, Seoul 07360 (KR); JEON, Yuna, Seoul 07622 (KR); BAI, Xuelian, Goyang-si Gyeonggi-do 10323 (KR); LIM, Saeyi, Seoul 07622 (KR); SONG, Hyerin, Seoul 07599 (KR)
(74) Representative: dompatent
(86) International application number: PCT/KR2024/018073
(87) International publication number: WO 2025/105860

(57) **Abstract**

The present invention relates to an SKP2 fragment, a variant thereof, and use thereof. Compared to a native SKP2 fragment, the SKP2 fragment variant according to the present invention has increased binding affinity to s-phase kinase associated protein 1 (SKP1). In addition, compared to a fusion protein comprising a native SKP2 fragment and a target binding site, a fusion protein comprising said SKP2 fragment variant and a target binding site can more effectively induce degradation of a target protein. Therefore, the SKP2 fragment variant of the present invention is expected to be usable in the development of PROTAC drug with improved therapeutic effect.

## Description

### TECHNICAL FIELD

The present invention relates to an SKP2 fragment and a variant thereof, and use thereof.

### BACKGROUND ART

Up to now, new drugs have been developed based on the principle of inhibiting the active site or ligand binding site of target proteins for diseases. Of the approximately 20,000 human proteins, only about 3% of proteins are known as FDA-approved drug targets, and about 3,100 proteins (16%), which correspond to most disease target proteins, are considered inapplicable to current new drug development technology. In addition, existing active site-directed new drugs often report problems with drug resistance.

A proteolysis-targeting chimera (PROTAC) is a dual-binding molecule that binds to a protein that causes a disease and induces protein hydrolysis via the proteasome. PROTAC can induce degradation of proteins that cannot be degraded by existing technologies by using the ubiquitin-proteasome system (UPS), which is an endogenous protein degradation mechanism that cells use to degrade proteins. Therefore, it is attracting attention as a new paradigm for overcoming the limitations of traditional new drug development.

PROTAC is composed of two protein binding molecules, which bind to E3 ubiquitin ligase and target protein, respectively. Through this binding, PROTAC brings the target protein close to the E3 ligase, thereby inducing ubiquitination. Ubiquitination involves three steps of activation, conjugation and ligation, which are performed by ubiquitin-activating enzyme (E1), ubiquitin-conjugating enzyme (E2), and ubiquitin ligase (E3). As a result of this cascade, ubiquitin is covalently bonded to the target protein, and the ubiquitinated protein is degraded by the proteasome.

The PROTAC technology was first introduced in 2001, and is currently receiving much attention and actively developing related technologies (Sakamoto et al., PNAS, 98:8554 (2001), Huang et al., Cell Res, 26:484-498 (2016)). Most PROTAC drugs have been developed as small-molecule compounds. Although known PROTAC drugs are very useful, improvements are needed to address the inherent low solubility of small-molecule compounds, low protein degradation efficiency due to decreased intracellular penetration, and safety concerns, and there is a demand for PROTAC drugs that improve these issues.

### DISCLOSURE

### TECHNICAL PROBLEM

Accordingly, the inventors of the present invention conducted research to develop a more effective PROTAC drug, discovered a variant of an SKP2 (S-phase kinase-associated protein 2) fragment, and confirmed that the variant forms a stronger E3 ubiquitin ligase complex compared to the wild type, thereby completing the present invention.

### TECHNICAL SOLUTION

In order to achieve the above object, one aspect of the present invention provides an SKP2 fragment or a variant thereof, and a fusion protein including the SKP2 fragment or variant thereof; and a target binding site.

Another aspect of the present invention provides a polynucleotide encoding the SKP2 fragment or variant thereof, or the fusion protein, a vector loaded with the polynucleotide, and a host cell transformed with the vector.

Still another aspect of the present invention provides a pharmaceutical composition for preventing or treating a disease, including the fusion protein, a polynucleotide encoding the fusion protein or a vector including the polynucleotide as an active ingredient.

Still another aspect of the present invention provides the use of the fusion protein, a polynucleotide encoding the fusion protein or a vector including the polynucleotide in the prevention or treatment of a disease.

Still another aspect of the present invention provides a method for preventing or treating a disease, including administering the fusion protein, a polynucleotide encoding the fusion protein or a vector including the polynucleotide to a subject.

### ADVANTAGEOUS EFFECTS

The variant of an SKP2 fragment according to the present invention has an increased binding affinity to SKP1 (S-phase kinase associated protein 1) compared to the wild-type SKP2 fragment. In addition, the variant of the SKP2 fragment and the fusion protein including a target binding site can induce the degradation of a target protein more effectively compared to the wild-type SKP2 fragment and the fusion protein including the target binding site. Therefore, it is expected that the variant of the SKP2 fragment of the present invention can be used to develop a PROTAC drug with improved therapeutic efficacy.

### DESCRIPTION OF DRAWINGS

FIGS. 1a to 1d are diagrams showing the results of confirming the binding of the SKP2₂₋₁₇₆ mutant protein library to SKP1.
FIGS. 2a to 2c are diagrams showing the results of confirming the binding of the secondary SKP2₂₋₁₇₆ mutant protein library to SKP1.
FIG. 3 is a diagram showing the amino acid sequences of variants selected from the first SKP2₂₋₁₇₆ mutant protein library (clone 3, clone 15) and the second SKP2₂₋₁₇₆ mutant protein library (clone E1, clone E2, clones E4 to E6).
FIGS. 4a to 4c are graphs showing the results of confirming the binding affinity of SKP2₂₋₁₇₆ and mutant proteins thereof (clone E1, clone E2, clone E4 to clone E6) to SKP1.
FIG. 5 is a diagram (top) showing the results of Western blot analysis of the degradation capability of a STAT3 protein of a fusion protein including SKP2₂₋₁₇₆ or a variant thereof and an anti-ALFA tag nanobody (NbALFA) as one specific example of the present invention, and a graph (bottom) showing the results of quantifying the results.
FIG. 6 is a diagram (top) showing the results of Western blot analysis of the STAT3 protein degradation capability of a fusion protein including SKP2₂₋₁₇₆ or a variant thereof and an anti-STAT3 antibody (ETI01_A4 antibody variant) as one specific example of the present invention, and a graph (bottom) showing the results of quantifying the results.

### BEST MODE

### SKP2 fragment and variant thereof

One aspect of the present invention provides an SKP2 fragment or a variant thereof.

As used herein, the term "SKP2 (S-phase kinase-associated protein 2)" is an F-box protein constituting the SCF^{Skp2} ubiquitin ligase (E3 ligase) complex. The SKP2 protein includes a total of 424 amino acids, and includes an F-box domain (94 aa to 140 aa) consisting of about 40 amino acids from the N-terminus and 10 LRRs (leucin rich regions).

F-box proteins are classified into three groups of Fbxws including a WD40 repeat domain, Fbxls including a leucin-rich repeat domain and Fbxos including other protein-protein interaction sites or no recognition motif. F-box proteins are one of the proteins constituting the SCF (SKP1-cullin-F-box) ubiquitin ligase complex and function as substrate recognition factors. Therefore, different substrate proteins may bind depending on the type of F-box. In addition, it binds to SKP1 through the F-box domain to form the SCF ubiquitin complex.

The SKP2 protein is an F-box protein belonging to Fbxls, which binds to cell cycle regulatory proteins such as p27 and p21 and regulates their degradation.

The SKP2 protein may preferably be a human SKP2 protein, but is not limited thereto. The amino acid sequence and polynucleotide sequence of the SKP2 protein may be obtained from known databases such as GenBank of the National Institutes of Health (NCBI) of the United States, and may preferably include an amino acid sequence of SEQ ID NO: 47.

In addition, it may be composed of a sequence in which one or several amino acids of the protein are added, deleted or substituted, as long as it has the same activity as the protein with respect to the SKP1 protein or the gene location encoding the SKP2 protein on the chromosome is the same. The SKP2 protein may comprise or consist of an amino acid sequence having about 90%, about 91%, about 92%, about 93%, about 94%, about 95%, about 96%, about 97%, about 98%, or about 99% identity or 100% identity with the amino acid sequence of SEQ ID NO: 47. Here, binding to SKP1 may be measured by a method known to those skilled in the art.

The term "SKP2 fragment" used in the present specification means a fragment in which a portion of the N-terminus and/or the C-terminus of the wild-type SKP2 protein is truncated, and may exhibit the same binding affinity to SKP1 as the wild-type SKP2 protein. The "wild type" includes all proteins found in nature or nucleic acids encoding the same, and may be described interchangeably with the wild type.

Specifically, the fragment may include a site that binds to SKP1. More specifically, it may include an F-box domain that binds to SKP1. In this case, the F-box domain is the same as described above.

More specifically, the SKP2 fragment may include a sequence of 139, 140, 141, 142, 143, 144, 145, 146, 147, 178, 149, 150, 151, 152, 153, 154, 155, 156, 157, 158, 159, 160, 161, 162, 163, 164, 165, 166, 167, 168, 169, 170, 171, 172, 173, 174 or 175 amino acids consecutively from the 2nd amino acid of the N-terminus of the protein having an amino acid sequence of SEQ ID NO: 47. In one specific example, the SKP2 fragment may comprise or consist of an amino acid sequence of SEQ ID NO: 1.

In addition, the SKP2 fragment may be composed of a sequence in which one or more amino acids of the fragment are added, deleted or substituted, as long as it has the same binding affinity to the SKP2 fragment for the SKP1 protein or has the same gene location encoding the SKP2 protein on the chromosome. Specifically, the SKP2 protein may comprise or consist of an amino acid sequence having about 90%, about 91%, about 92%, about 93%, about 94%, about 95%, about 96%, about 97%, about 98%, or about 99% identity or 100% identity with an amino acid sequence of SEQ ID NO: 1.

The term "SKP2 fragment variant" used herein refers to a form in which a portion of the amino acid of the SKP2 fragment described above is substituted. That is, the SKP2 fragment variant refers to a peptide having a different sequence from the wild-type SKP2 fragment and having the function of binding to SKP1. In this case, the SKP2 fragment variant may exhibit binding affinity for binding to SKP1 that is about 10 times to about 650 times improved compared to the wild-type SKP2 fragment. In this case, "binding affinity" refers to the strength of a sum total of non-covalent interactions between a single binding site of a molecule and its binding partner.

Here, binding affinity to SKP1 may be measured by methods known to those skilled in the art.

Specifically, the SKP2 fragment variant may have 7 to 14 amino acids substituted in the wild-type SKP2 fragment.

More specifically, the SKP2 fragment variant may be substituted with any one amino acid selected from the group consisting of 2nd, 4th, 11th, 12th, 19th, 24th, 29th, 33rd, 44th, 59th, 78th, 98th, 102nd, 107th, 111th, 112th, 113th, 124th, 128th, 130th, 133rd, 135th, 137th, 138th, 139th, 148th, 156th, 161st, 176th amino acids and a combination thereof in an amino acid sequence of SEQ ID NO: 1.

More specifically, the variant may be substituted with any one amino acid selected from the group consisting of H2, K4, D11, L12, S19, W24, T29, L33, E44, L59, D78, D98, D102, G107, C111, L112, C113, C124, Y128, L130, D133, S135, W137, Q138, T139, H148, L156, I161, E176 and a combination thereof in an amino acid sequence of SEQ ID NO: 1.

In this case, the "amino acid" introduced by the substitution may be any one selected from the group consisting of glycine, alanine, valine, leucine, isoleucine, serine, threonine, cysteine, methionine, aspartic acid, glutamic acid, asparagine, glutamine, lysine, arginine, phenylalanine, tyrosine, tryptophan, histidine and proline.

More specifically, the variant may be substituted with an amino acid selected from the group consisting of H2R, K4R, D11G, L12P, S19G, W24R, T29A, L33P, E44K, L59P, D78G, D98V, D102N, G107R, C111R, L112P, L112R, C113R, C124S, Y128C, L130Q, D133E, S135P, W137R, Q138K, T139S, H148R, L156Q, L156R, I161A, I161V, E176G and a combination thereof in an amino acid sequence of SEQ ID NO: 1. In this case, when L112 is substituted, it may be substituted with L112P or L112R. When L156 is substituted, it may be substituted with L156Q or L156R. When I161 is substituted, it may be substituted with I161A or I161V.

As a specific example, the SKP2 fragment variant may comprise or consist of any one amino acid sequence selected from the group consisting of SEQ ID NO: 2 to SEQ ID NO: 8.

### Fusion protein

Another aspect of the present invention provides a fusion protein including an SKP2 fragment or a variant thereof; and a target protein binding site. In this case, the SKP2 fragment and the variant thereof are the same as described above.

As used herein, the term "target protein" means a protein that is specifically expressed in a target (intended) cell, tissue or disease environment or that can cause a disease.

The target protein may be a protein present on a cell surface or within a cell. For example, the target protein may include a structural protein, a receptor, an enzyme, a cell surface protein and the like. The target protein may be, for example, any peptide or small molecule that binds to: FoxOl, HDAC, DP-1, E2F, ABL, AMPK, BRK, BRSK I, BRSK2, BTK, CAMKK1, CAMKK alpha, CAMKK beta, Rb, Suv39HI, SCF, p19INK4D, GSK-3, pi8 INK4, myc, cyclin E, CDK2, CDK9, CDG4/6, cyclin D, pl6 INK4A, cdc25A, BMI1, SCF, Akt, CHKl/2, C 1 delta, CK1 gamma, C2, CLK2, CSK, DDR2, DYRK1A/2/3, EF2K, EPH-A2/A4/B 1/B2/B3/B4, EIF2A 3, Smad2, Smad3, Smad4, Smad7, p53, p21 Cipl, PAX, Fyn, CAS, C3G, SOS, Tal, Raptor, RACK-1, CRK, Rapl, Rac, KRas, NRas, HRas, GRB2, FAK, PI3K, spred, Spry, mTOR, MPK, LKBl, PAK 1/2/4/5/6, PDGFRA, PYK2, Src, SRPK1, PLC, PKC, PKA, PKB alpha/beta, PKC alpha/gamma/zeta, PKD, PLKl, PRAK, PRK2, WAVE-2, TSC2, DAPKI, BAD, IMP, C-TAK1, TAKl, TAOl, TBK1, TESK1, TGFBR1, TIE2, TLK1, TrkA, TSSK1, TTBK1/2, TTK, Tpl2/cotl, MEK1, MEK2, PLDL Erk1, Erk2, Erk5, Erk8, p90RSK, PEA-15, SRF, p27 KIP1, TIF la, HMGN1, ER81, MKP-3, c-Fos, FGF-R1, GCK, GSK3 beta, HER4, HIPK1/2/3/, IGF-1R, cdc25, UBF, LAMTOR2, Statl, Stat3, StaO, CREB, JAK, Src, PTEN, NF-kappa B, HECTH9, Bax, HSP70, HSP90, Apaf-1, Cyto c, BCL-2, Bcl-xL, Smac, XIAP, Caspase-9, Caspase-3, Caspase-6, Caspase-7, CDC37, TAB, IKK, TRADD, TRAF2, R1P1, FLIP, TAKl, JNKl/2/3, Lck, A-Raf, B-Raf, C-Raf, MOS, MLKl/3, MN 1/2, MSKl, MST2/3/4, MPSK1, MEKKI, ME K4, MEL, ASK1, MINK1, MKK 1/2/3/4/6/7, NE 2a/6/7, NUAK1, OSR1, SAP, STK33, Syk, Lyn, PDK1, PHK, PIM 1/2/3, Ataxin-1, mTORCl, MDM2, p21 Wafl, Cyclin D1, Lamln A, Tpl2, Myc, catenin, Wnt, IKK-beta, IKK-gamma, IKK-alpha, IKK-epsilon, ELK, p65RelA, IRAKI, IRA 2, IRAK4, IRR, FADD, TRAF6, TRAF3, MKK3, MKK6, ROCK2, RSK1/2, SGK 1, SmMLCK, SIK2/3, ULK1/2, VEGFR1, WNK l, YES1, ZAP70, MAP4K3, MAP4K5, MAPKlb, MAPKAP-K2 K3, p38 alpha/beta/delta/gamma MAPK, Aurora A, Aurora B, Aurora C, MCAK, Clip, MAPKAPK, FAK, MARK 1/2/3/4, Mucl, SHC, CXCR4, Gap-1, Myc, beta-catenin/TCF, Cbl, BRM, Mcl-1, BRD2, BRD3, BRD4, AR, RAS, ErbB3, EGFR, IRE1, HPK1, RIPK2, ERct and variants thereof.

As used herein, the term "target protein binding site" means a site that binds to a target protein. Specifically, it may be a ligand (peptide), antibody, partner binding protein or the like that binds to a target protein. The antibody may include an antibody fragment. The antibody fragment may include a Fab fragment, a Fab' fragment, an F(ab')₂ fragment, a bispecific Fab dimer (Fab2), a trispecific Fab trimer (Fab₃), Fv, a single-chain Fv protein (scFv), bis-scFv (scFv)₂, a minibody, a diabody, a triabody, a tetrabody, a disulfide-stabilized Fv protein (dsFv), a single-domain antibody (sdAb, nanobody), a heavy-chain only antibody (e.g., camelid VHH, camelid nanobody, shark Ig NAR), an affibody, an intrabody and the like. Specifically, it may be a single domain antibody.

The term "single domain antibody (sdAb)" used in the present specification refers to a nanobody, which is an antibody fragment composed of a single variable region fragment of an antibody. sdAb derived primarily from heavy chains is used, but single variable region fragments derived from light chains are also reported to specifically bind to antigens.

In one specific example, the target binding protein may be a nanobody that binds to Stat3. Specifically, the target binding protein may include an amino acid sequence of SEQ ID NO: 65.

The SKP2 fragment or variant thereof and the target protein binding portion may be linked via a linker. In this case, the linker may be a peptide linker.

Specifically, the fusion protein may consist of Structural Formula (I) or (II) below.

N'-TB-(L)n-SR-C' (I)

N'-SR-(L)n-TB-C' (II)

In this case, in Structural Formulas (I) and (II),
the N' is an N-terminus of the fusion protein,
the C' is a C-terminus of the fusion protein,
the TB is a target protein binding site (target binder),
the SR is an SKP2 fragment or a variant thereof,
the L is a peptide linker, and
the n is 0 or 1.

In this case, the target protein binding site and the SKP2 fragment or variant thereof are the same as described above.

The peptide linker may consist of 1 to 30 consecutive amino acids, or 2 to 20 consecutive amino acids, or 2 to 10 amino acids. In one specific example, the peptide linker may be (GS)n (where n is an integer from 1 to 10). In this case, n in (GS)n may be 1, 2, 3, 4, 5, 6, 7, 8, 9 or 10. The peptide linker may use, without particular limitation, a sequence linker known in the art, such as GS, GGGGS, (GGGGS)₂, ASTKGP, ASTKGPSVFPLAP or the like, which provides structural flexibility without being cleaved by a proteolytic enzyme. In one embodiment, the peptide linker may consist of an amino acid sequence of SEQ ID NO: 48 (GS).

### Polynucleotide encoding SKP2 fragment or variant thereof, or fusion protein

Still another aspect of the present invention provides a polynucleotide encoding an SKP2 fragment or a variant thereof, or the fusion protein. The SKP2 fragment or a variant thereof, and the fusion protein are the same as described above.

Specifically, the polynucleotide encoding the SKP2 fragment may include a base sequence of SEQ ID NO: 23. In addition, the polynucleotide encoding a variant of the SKP2 fragment may include any one base sequence selected from the group consisting of SEQ ID NOs: 24 to 30.

The polynucleotide encoding the fusion protein may include a base sequence encoding a target protein binding site and an SKP2 fragment or a variant thereof. In one specific example, the target protein binding site may include a base sequence of SEQ ID NO: 66. Accordingly, in one specific example, the polynucleotide encoding the fusion protein may include any one base sequence selected from the group consisting of SEQ ID NO: 57 to SEQ ID NO: 64.

In addition, the polynucleotide may be mutated by substitution, deletion, insertion or a combination thereof of one or more bases, as long as it encodes the same polypeptide. When the polynucleotide sequence is prepared by chemical synthesis, synthetic methods well known in the art may be used, for example, the method described in the literature (Engels and Uhlmann, Angew Chem IntEd Engl., 37:73-127, 1988), and examples thereof include triester, phosphite, phosphoramidite and H-phosphate methods, PCR and other autoprimer methods, and oligonucleotide synthesis methods on solid supports.

Specifically, the polynucleotide may include a nucleic acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99% or at least about 100% identity to the base sequences of SEQ ID NOs: 23 to 30 and SEQ ID NOs: 57 to 64, respectively.

### Vector loaded with polynucleotide

Still another aspect of the present invention provides a vector loaded with a polynucleotide encoding an SKP2 fragment or a variant thereof, or the fusion protein. In this case, the polynucleotide encoding the SKP2 fragment may include a base sequence of SEQ ID NO: 23. In addition, the polynucleotide encoding the variant of the SKP2 fragment may include any one base sequence selected from the group consisting of SEQ ID NOs: 24 to 30. As a specific example of the polynucleotide encoding the fusion protein, the polynucleotide may include any one base sequence selected from the group consisting of SEQ ID NOs: 57 to 66. The SKP2 fragment or variant thereof and the fusion protein are the same as described above.

As used herein, the term "vector" may be introduced into a host cell and recombined and inserted into the host cell genome. Alternatively, the vector is understood to be an episome capable of autonomously replicating a nucleotide sequence. The vector includes linear nucleic acids, plasmids, phagemids, cosmids, RNA vectors, viral vectors, mini-chromosomes and analogs thereof. Examples of viral vectors include, but are not limited to, retroviruses, adenoviruses and adeno-associated viruses.

Specifically, the vector may be a plasmid DNA, a phage DNA and the like, and may be a commercially developed plasmid (e.g., pUC18, pBAD, pIDTSAMRT-AMP, etc.), an *E. coli*-derived plasmid (e.g., pYG601BR322, pBR325, pUC118, pUC119, etc.), a *Bacillus subtilis*-derived plasmid (e.g., pUB110, pTP5, etc.), a yeast-derived plasmid (e.g., YEp13, YEp24, YCp50, etc.), a phage DNA (e.g., Charon4A, Charon21A, EMBL3, EMBL4, λgt10, λgt11, λZAP, etc.), an animal virus vector (e.g., retrovirus, adenovirus, vaccinia virus, etc.), an insect virus vector (baculovirus, etc.) and the like. The vector shows different protein expression levels and formulas depending on the host cell, and thus, it is desirable to select and use the host cell most suitable for the purpose.

The vector of the present invention may be fused with other sequences to facilitate purification of antibodies expressed therefrom. Sequences to be fused include, for example, FLAG (IBI, USA), glutathione S-transferase (Pharmacia, USA), maltose binding protein (NEB, USA) and 6X His (hexahistidine; Quiagen, USA).

In addition, since the protein expressed by the vector of the present invention is an antibody, the expressed antibody may be easily purified through a protein A column or the like without an additional sequence for purification.

### Transformed cell

Still another aspect of the present invention provides a transformed host cell into which an expression vector including a polynucleotide encoding an SKP2 fragment or a variant thereof, or the fusion protein, is introduced.

The term "transformed host cell" as used herein refers to prokaryotic cells and eukaryotic cells into which a recombinant expression vector can be introduced. The transformed cell may be produced by introducing a vector into a host cell and transforming the same. In addition, the polynucleotide included in the vector may be expressed to produce the SKP2 fragment of the present invention or a variant thereof, or the fusion protein.

The transformation may be performed by various methods. It is not particularly limited thereto, as long as it can produce the SKP2 fragment of the present invention or a variant thereof, or the fusion protein. Specifically, for the transformation method,a CaCl₂ precipitation method, a Hanahan method with increased efficiency by using a reducing substance called dimethyl sulfoxide (DMSO) in the CaCl₂ precipitation method, electroporation, a calcium phosphate precipitation method, a protoplast fusion method, a stirring method using a silicon carbide fiber, an *Agrobacterium*-mediated transformation method, a transformation method using PEG, dextran sulfate, lipofectamine and a drying/inhibition-mediated transformation method may be used. In addition, a target substance may be delivered into a cell using a virus particle by infection. In addition, a vector may be introduced into a host cell by gene bombardment and the like.

In addition, the host cell used for producing the transformed cell is also not particularly limited thereto, as long as it can produce the antibody of the present invention. Specifically, the host cell may include, but is not limited to, a prokaryotic cell, a eukaryotic cell, a mammal, a plant, an insect, a fungus or a cell of cellular origin. An example of the prokaryotic cell may be *Escherichia coli*. In addition, an example of the eukaryotic cell may be yeast. In addition, as the mammalian cell, CHO cells, F2N cells, COS cells, BHK cells, Bowes melanoma cells, HeLa cells, 911 cells, AT1080 cells, A549 cells, SP2/0 cells, human lymphoblastoid, NSO cells, HT-1080 cells, PERC.6 cells, HEK293 cells or HEK293T cells may be used, but is not limited thereto, and any cell known to those skilled in the art that can be used as a mammalian host cell may be used.

Additionally, in order to optimize the therapeutic properties of the antibody or for other purposes, the glycosylation-related genes of the host cell may be manipulated by methods known to those skilled in the art to adjust the sugar chain pattern of the antibody (e.g., sialic acid, fucosylation, glycosylation).

### Pharmaceutical composition including fusion protein, polynucleotide encoding fusion protein or vector including polynucleotide

Still another aspect of the present invention provides a pharmaceutical composition for preventing or treating a disease, including a fusion protein including an SKP2 fragment or a variant thereof and a target protein binding site, a polynucleotide encoding the fusion protein or a vector including the polynucleotide as an active ingredient.

The above terms, "fusion protein", "polynucleotide encoding fusion protein" and "vector including the polynucleotide" are the same as described above.

The polynucleotide may be DNA, mRNA, plasmid DNA and the like, and the polynucleotide or a vector including the same may be delivered into a cell by a cell-penetrating functional nano-carrier to exhibit the same target protein degradation efficacy as the fusion protein.

The disease may be selected from the group consisting of cancer, stroke, ischemic disease, peripheral vascular disease, alcoholic liver disease, hepatitis, cirrhosis, Parkinson's disease, Alzheimer's disease, fibrosis, diabetes, ALS, pathogenic disease, inflammatory disease, arthritis, anemia, genetic disorder, hyperglycemia, metabolic syndrome, lipodystrophy syndrome, dyslipidemia, insulin resistance, leptin resistance, atherosclerosis, vascular disease, hypercholesterolemia, hypertriglyceridemia, nonalcoholic fatty liver disease and obesity.

The term "treatment" as used herein may be used to mean both therapeutic treatment and preventive treatment, and includes any application or form of medication for treating a disease in mammals, including humans. In addition, the term includes inhibiting or slowing the progression of a disease; restoring or repairing damaged or defective functions, thereby partially or completely alleviating a disease; or stimulating an ineffective process; or alleviating a serious disease. The term "prevention" may be used to mean alleviating or reducing a pathological condition or disease of a subject.

In the present invention, the nucleic acid may be used together with various carriers such as lipid nanoparticles (LNPs), liposomes or vesicles, which are known to effectively deliver polynucleotides into cells, but the present invention is not limited thereto.

In the present invention, the pharmaceutical composition may be administered systemically via parenteral administration. Parenteral administration may be administered in the form of nasal, intranasal, oral, intravenous, intramuscular, intraarterial, intramedullary, intrathecal, intracardiac, transdermal, subcutaneous, intradermal, intraperitoneal, enteral, topical, sublingual or rectal administration, but is not limited thereto.

The pharmaceutical composition may be prepared in the form of, for example, a powder, a tablet, a capsule, a liquid, an ointment, a cream, a gel, a hydrogel, an aerosol, a spray, a micellar solution, a transdermal patch, a liposomal suspension, a polyplex, an emulsion, a lipid nanoparticle (LNP) (having RNA on its surface or encapsulated therein) or any other suitable form, which can be administered to a human or mammal in need of treatment.

Still another aspect of the present invention provides the use of the fusion protein, a polynucleotide encoding the fusion protein, or a vector including the polynucleotide, or a pharmaceutical composition including the same in the prevention or treatment of a disease.

Still another aspect of the present invention provides a method for preventing or treating a disease, including administering the fusion protein, a polynucleotide encoding the fusion protein, a vector including the polynucleotide, or a pharmaceutical composition including the same to a subject.

The fusion protein, the polynucleotide encoding the same, the vector including the polynucleotide and the pharmaceutical composition are the same as described above.

### MODES OF THE INVENTION

Hereinafter, the present invention will be described in detail by the following examples. However, the following examples are only intended to illustrate the present invention, and the present invention is not limited thereto.

### Preparation Example 1. Preparation of anti-STAT3 nanobody

A gene encoding the anti-STAT3 nanobody VHH13 (referred to as SBT-100; see US9,695,234) was synthesized (IDT Technology) and inserted into pYD5, a yeast surface expression vector, which was constructed with reference to a research article (Wang Z et al. "A new yeast display vector permitting free scFv amino termini can augment ligand binding affinities". Protein Eng Des Sel. (2005) 18:337-343, doi:10.1093/protein/gzi036). Thereafter, the CDR of the plasmid was divided into 5 regions (H1, H2A, H2B, H3A, H3B), and primers were synthesized (IDT Technology) to induce random mutations in the regions, and PCR was performed by using these primers. The final 5 ug of randomly mutated PCR product was inserted into 1 ug of pYD5 vector and transformed into EBY100 (ATCC, Cat no. MYA-4941) yeast to construct a library with a diversity of approximately 0.5 × 10⁷ to 1.0 × 10⁷.

By using the above library, ETI01_A4 nanobody with high binding affinity to STAT3 was prepared. The specific sequence of ETI01_A4 is as follows.

**[Table 1]**

| SEQ ID NO: | Name | Type | Length | Sequence |
|---|---|---|---|---|
| 65 | ETI01_A4 | PRT | 127aa | HVQLVESGGGSVQAGGSLRLSCAASGFNSGDSCIGW FRQVPGKEREGVSVISSGGEIRYYADSVKGRFTISQDN TKNTLYLQMNSLKPEDTAMYYCATSVFLCYPRTWF NRYMYNSWGQGTQVTVSS |
| 66 | ETI01_A4 polynucleot ide | DNA | 381bp | CATGTTCAGCTTGTAGAAAGTGGGGGAGGCTCCGT TCAAGCTGGTGGGAGCCTTCGGCTGTCTTGTGCAGC GAGCGGTTTTAACTCGGGTGATAGTTGCATTGGTTG GTTCCGACAGGTTCCGGGTAAAGAGCGAGAGGGCG TAAGTGTGATTTCTTCCGGCGGGGAGATTAGGTATT ATGCTGATAGTGTTAAGGGACGCTTTACAATCTCTC AGGATAATACCAAAAACACCCTGTATTTGCAAATG AACTCTCTTAAGCCTGAAGATACGGCAATGTACTA CTGCGCCACCTCCGTTTTTCTGTGTTACCCGAGGAC GTGGTTCAATAGGTATATGTATAACTCATGGGGTCA GGGAACCCAGGTAACGGTCAGTAGC |

### Example 1. Construction of primary library for SKP2 2-176 mutant protein

A gene encoding amino acids 2 to 176 (SKP2_{2-176,} SEQ ID NO: 1) of S-phase Kinase Associated Protein-2 (Uniprot ID: Q13309) was synthesized (IDT Technology) and inserted into the yeast surface expression vector pYD5. PCR was performed by using the above plasmid as a template using a Diversify PCR Random mutagenesis kit (Clonetech, Cat no. 630703) to induce 8.1 random mutations per 1,000 bp. The final 2.5 ug of randomly mutated PCR products were each inserted into 0.5 ug of the pYD5 vector. EBY100 (ATCC, Cat no. MYA-4941) yeast was transformed with each of the above vectors to construct a library with 6.0 × 10⁶ diversity.

### Example 2. Binding affinity and single-clone analysis of SKP2 2-176 mutant protein library to SKP1

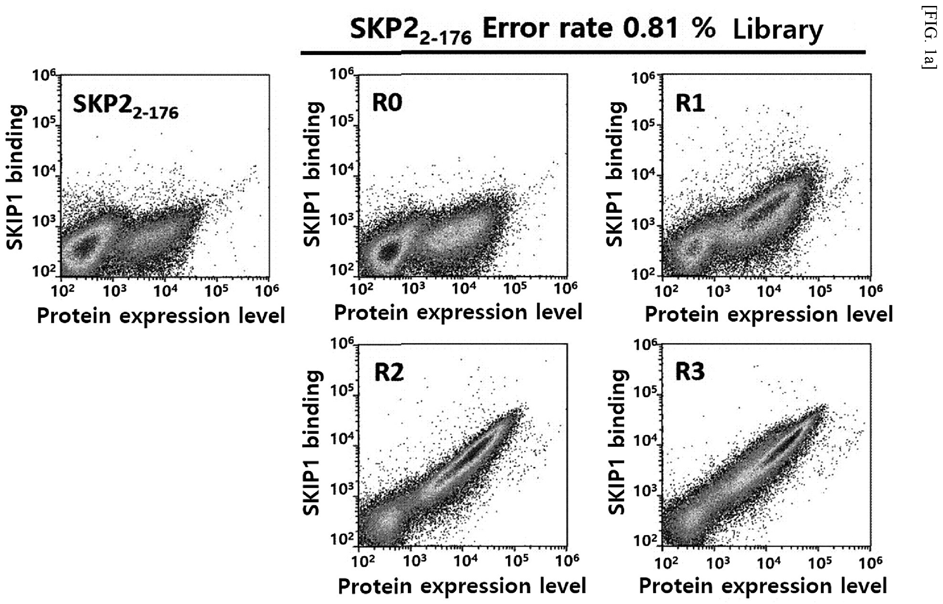

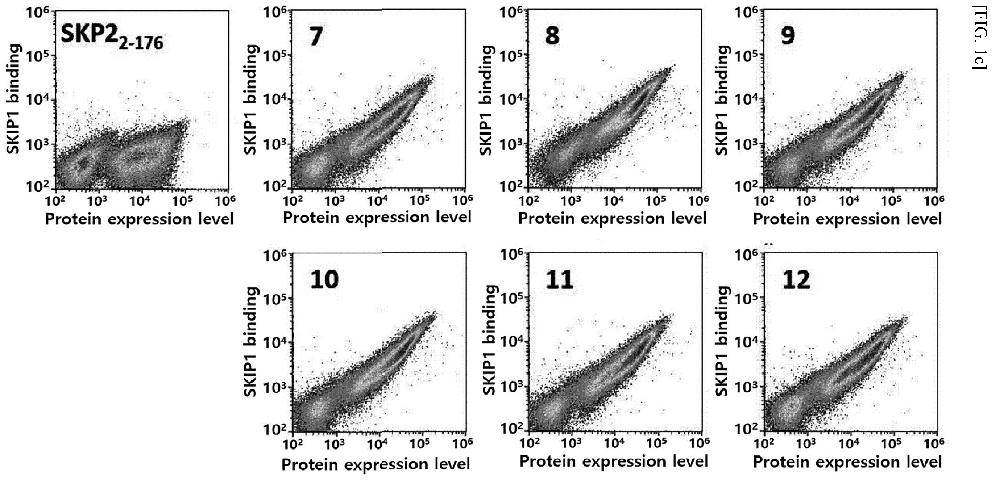

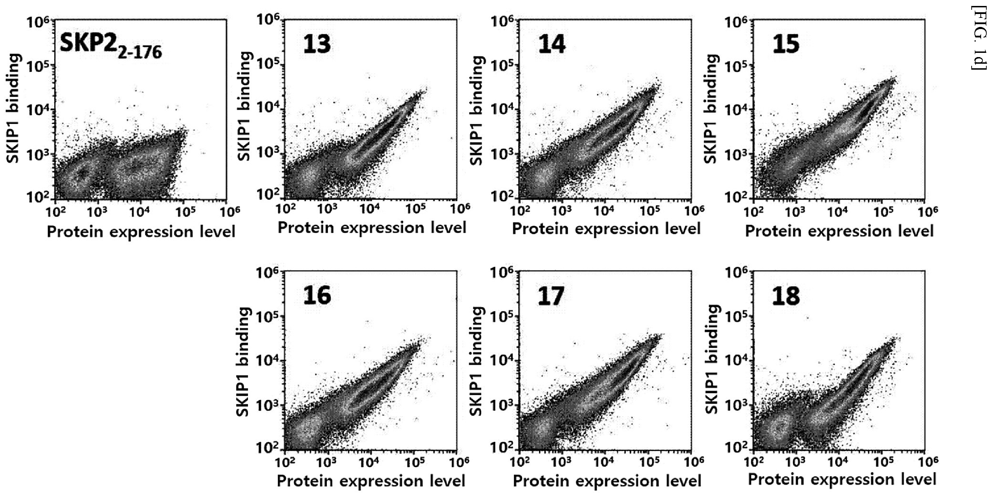

The constructed primary library was cultured at 30°C for approximately 16 hours by using SD-CAA medium (including 20 g glucose (SIGMA, Cat no. G7528), 14.7 g sodium citrate (SIGMA, Cat no. C8532), 4.3 g citric acid monohydrate (SIGMA, Cat no. C0706), 6.7 g yeast nitrogen base (BD Difco, Cat no. 291940), 5 g bacto casamino acid (BD Difco, Cat no. 223050), and 100 ug/mL kanamycin (Biosesang, Cat no. KC1001-025-02) per 1 L of distilled water). After the culturing was completed, the culture was diluted in SG-CAA medium until the OD₆₀₀ value was 1, and it was re-cultured at 30°C for 18 hours to induce the expression of SKP2₂₋₁₇₆ mutant protein on the yeast surface. 0.5 × 10⁷ cultured yeast cells were mixed with 500 nM biotinylated SKP1 protein (Sino biological, Cat no. 14161-H40E-B) and mouse anti-V5 antibody (Invitrogen, Cat no. R960-25) with 0.1% BSA (BOVOGEN, Cat no. BSAS0.1) (1:500 diluted in wash buffer including PBS pH7.4 (Biosesang, Cat no. PR2004-100-74)) and reacted at room temperature for 30 minutes to perform primary staining. Secondary staining was performed with Streptavidin, R-Phycoerythrin Conjugate (SAPE) (Invitrogen, Cat no. SA10044) and anti-mouse IgG (H + L)-FITC antibody (Invitrogen, Cat no. 11-4011-85) diluted in wash buffer at a ratio of 1:100 and incubated at 4°C for 20 minutes. The stained reactants were analyzed by flow cytometry (SONY, SH800S) to isolate the top 0.1% of yeast populations with enhanced binding affinity for SKP1 compared to SKP2₂₋₁₇₆. After repeating the above process three times, the binding affinity of the SKP2₂₋₁₇₆ mutant protein library to SKP1 was determined from Round 0 to the final Round 3 (FIG. 1a).

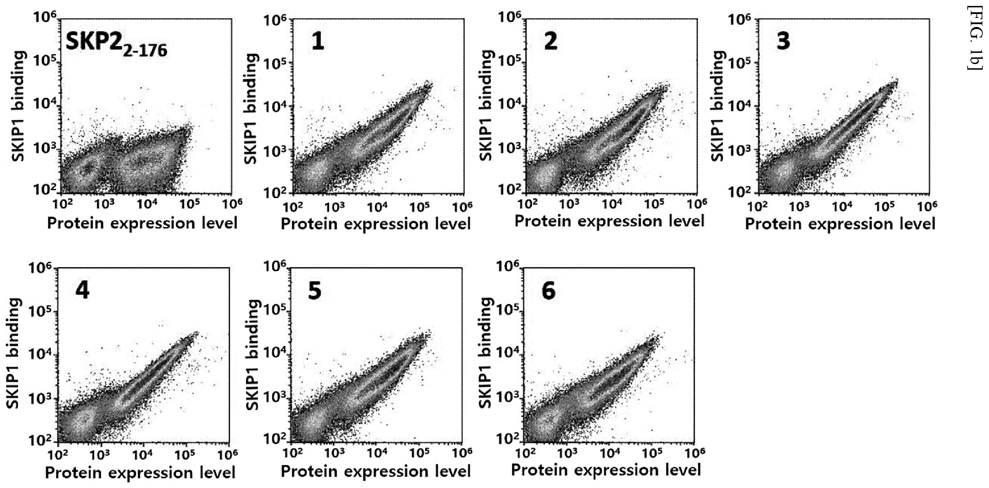

From the yeast population isolated after the last round, a single clone was analyzed under the condition of a biotinylated SKP1 protein concentration of 250 nM using the same method as above (FIGS. 1b to 1d). Among the single clones, clones '3' (SEQ ID NO: 2) and '15' (SEQ ID NO: 3) were selected from the clones with high binding affinity to SKP1, and the second random mutation was created by using the sequences of the two types of clones.

### Example 3. Construction of secondary library for SKP2 2-176 mutant protein

By using plasmids each having the sequences of clone '3' and clone '15' selected through the method of Example 2 as templates, PCR was performed in the same manner as Example 1 to induce 3.5 and 5.8 mutations per 1,000 bp. The final 2.5 ug of randomly mutated PCR products were inserted into 0.5 ug of pYD5 vector, and EBY100 yeast was transformed with each vector to construct 4 types of libraries, each having a diversity of 7.0 × 10⁶ to 9.0 × 10⁶.

### Example 4. Binding affinity and single-clone analysis of SKP2 2-176_3 and SKP22-176_15 mutant libraries to SKP1

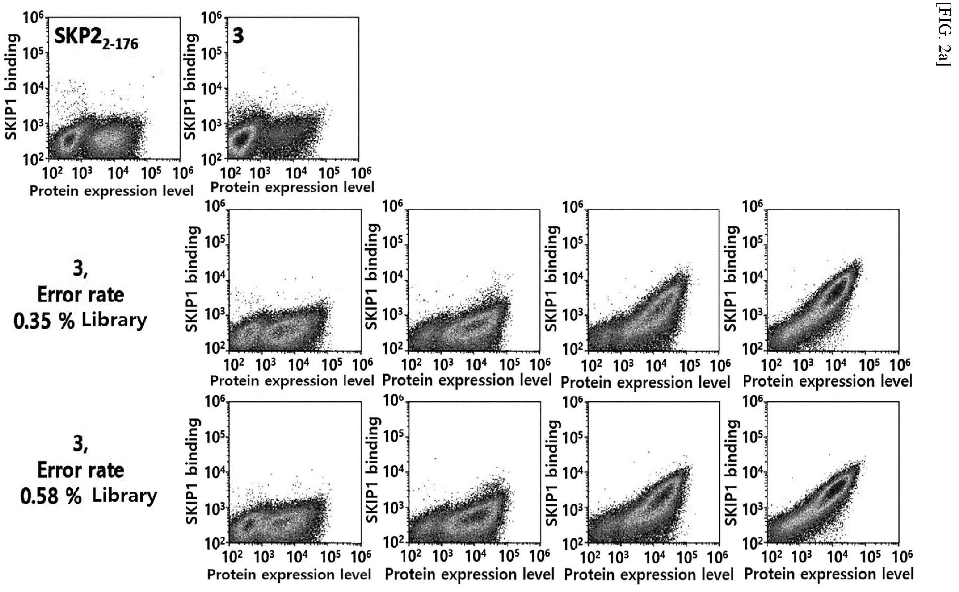

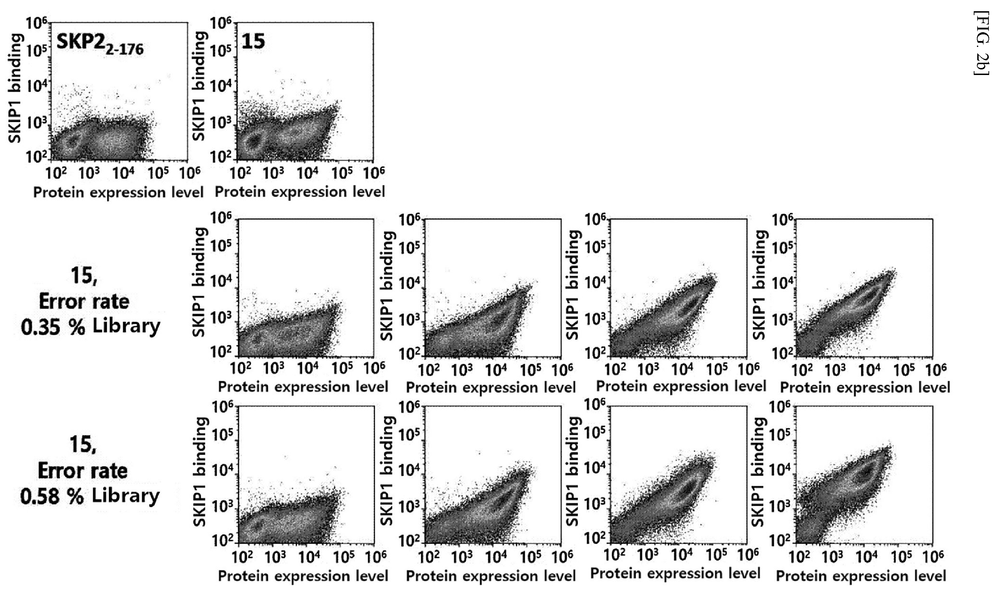

The constructed library was cultured in the same manner as in Example 2 to induce the expression of SKP2₂₋₁₇₆ mutant protein on the yeast surface. The cultured yeast (0.5 × 10⁷) was mixed with 5 nM biotinylated SKP1 and mouse anti-V5 antibody diluted in wash buffer at a ratio of 1:500, and reacted at room temperature for 30 minutes to perform primary staining. For secondary staining, Streptavidin, R-Phycoerythrin Conjugate (SAPE) and anti-mouse IgG (H + L)-FITC antibody diluted in wash buffer at a ratio of 1:100 were reacted in the dark at 4°C for 20 minutes. The reactants were used for flow cytometry to isolate individuals within the top 0.1% of the yeast population that had improved binding affinity for SKP1 compared to the SKP2₂₋₁₇₆_3 clone and SKP2₂₋₁₇₆_15 clone. After repeating the above process three times, the change in binding affinity of the SKP2₂₋₁₇₆ mutant protein library to SKP1 was confirmed from Round 0 to the final Round 3 (FIG. 2a and FIG. 2b).

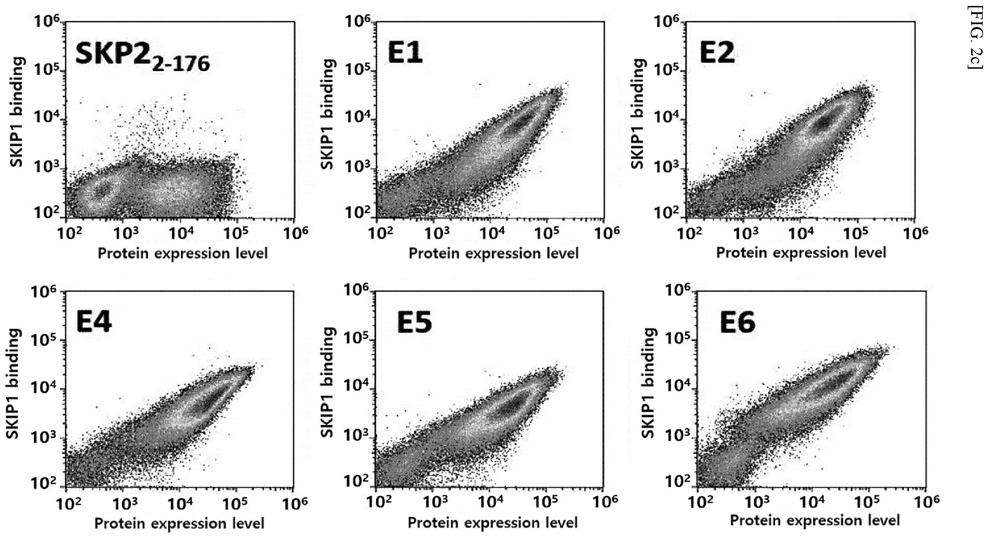

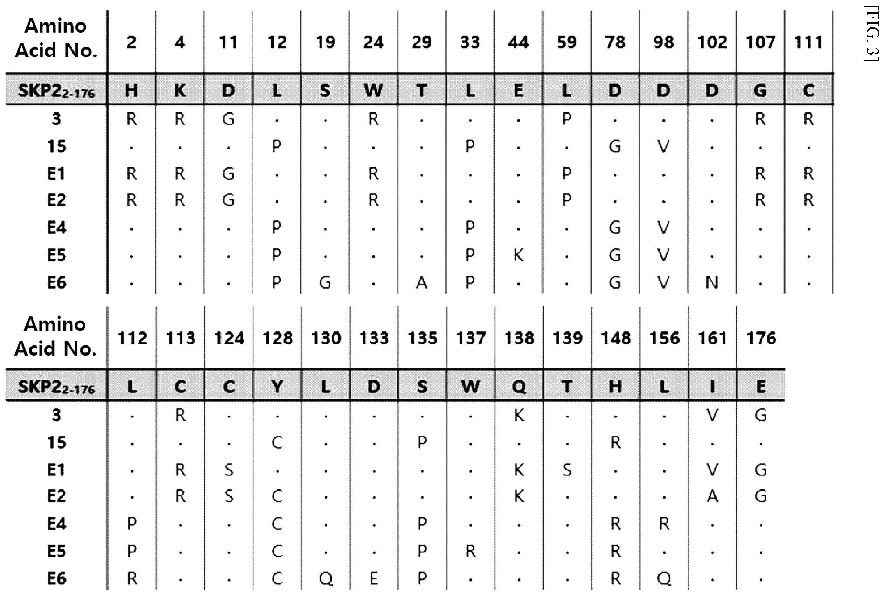

After the final round, the yeast individual clones separated were analyzed using the above method, and among the clones with high binding affinity, clones E1 (SEQ ID NO: 4), E2 (SEQ ID NO: 5), E4 (SEQ ID NO: 6), E5 (SEQ ID NO: 7) and E6 (SEQ ID NO: 8) were finally selected (FIG. 2c), and the sequences of each of the clones are shown in FIG. 3.

### Example 5. Binding affinity analysis of selected SKP2 2-176 mutant proteins to SKP1

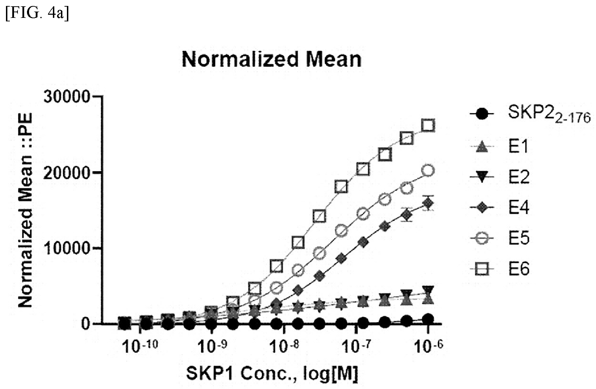

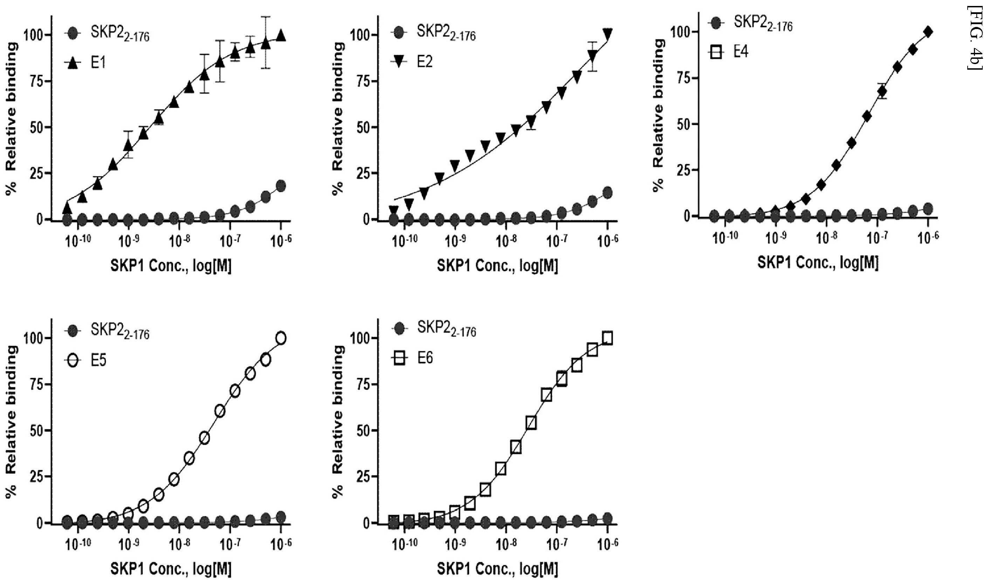

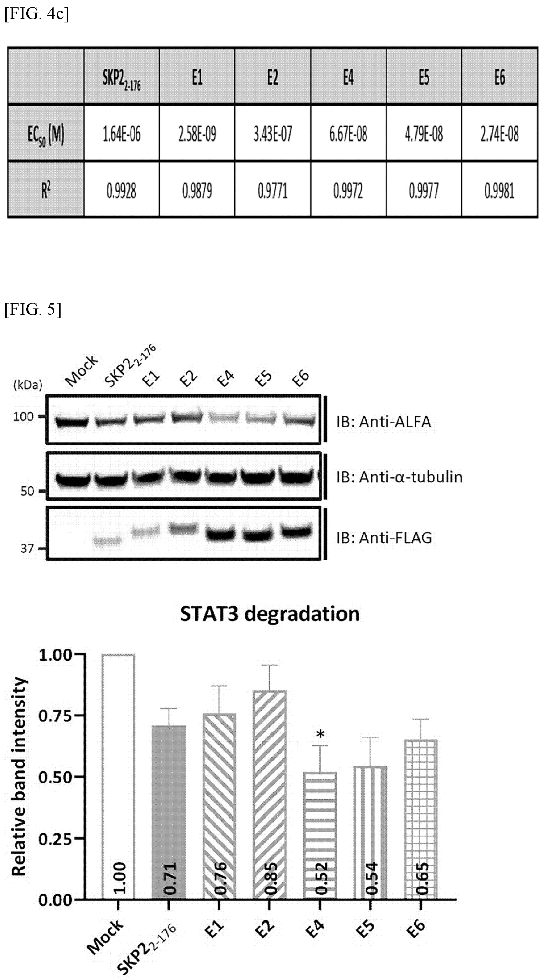

In order to compare the binding affinities of SKP2₂₋₁₇₆ and each variant, EC₅₀ was measured by using a flow cytometer. 6 final mutants (E1, E2, E4, E5 and E6) were induced to be expressed on the yeast surface in the same manner as in Example 2. 0.5 × 10⁷ cultured yeasts were mixed with mouse anti-V5 antibody at a ratio of 1:500, and 16 concentrations of biotinylated SKP1 serially diluted by 1/2 from 0 nM to 1000 nM, and the mixture was reacted for 30 minutes at room temperature to perform primary staining. For secondary staining, Streptavidin, R-Phycoerythrin Conjugate (SAPE) and anti-mouse IgG (H + L)-FITC antibody were diluted in wash buffer at a ratio of 1:100, and the mixture was reacted in the dark at 4°C for 20 minutes. The above reaction mixture was used to measure the binding pattern of the final clones to biotinylated SKP1 concentrations by using a flow cytometer (SONY, SH800S), and the results were analyzed by using the flow-jo program to derive the EC ₅₀ (FIG. 4a to FIG. 4c).

### Preparation Example 2. Preparation of expression vector for confirmation of target protein degradation capability

In order to evaluate the target protein degradation capability of SKP2₂₋₁₇₆ and variants thereof, the inventors of the present invention referred to a previous research article ("The ALFA-tag is a highly versatile tool for nanobody-based bioscience applications" Nat. Commun. 10:4403 (2019)). Specifically, an expression vector of a fusion protein (SKP2.GS.NbALFA), in which SKP2₂₋₁₇₆ or variants thereof and anti-ALFA tag nanobody (NbALFA) are linked via a GS linker (GS, SEQ ID NO: 48), and a plasmid in which the ALFA tag is conjugated to STAT3 (RefSeq. NM_139276.2, 2358 bp) were constructed.

More specifically, the polynucleotides encoding 'Flag tag-SKP2₂₋₁₇₆ .GS.NbALFA' (SEQ ID NO: 9) and 'Flag tag-SKP2₂₋₁₇₆ variants (E1, E2, E4, E5, E6).GS.NbALFA' (SEQ ID NOs: 10 to 14) including a Flag tag were respectively inserted from the N-terminus to the C-terminus in the pCMV6 (Origene, Cat no. PS100001) vector for expression in mammalian cells after restriction enzyme treatment. The STAT3 expression vector (ALFA-Myc tag.STAT3, SEQ ID NO: 15) was constructed by adding ALFA tag and Myc tag to the N-terminus using pCMV3_STAT3 (Sinobiological., Cat no. HG10034-NM, SEQ ID NO: 16).

**[Table 2]**

| SEQ ID NO: | Name | Amino acid sequence |
|---|---|---|
| 9 | Flag tag-SKP2(2-176).GS.NbALFA | DYKDDDDKTRHRKHLQEIPDLSSNVATSFTWGWDSSKTSELLS GMGVSALEKEEPDSENIPQELLSNLGHPESPPRKRLKSKGSDKD FVIVRRPKLNRENFPGVSWDSLPDELLLGIFSCLCLPELLKVSGV CKRWYRLASDESLWQTLDLTGKNLHPDVTGRLLSQGVIAFRCP |
| | | RSFMDQPLAEGSEVQLQESGGGLVQPGGSLRLSCTASGVTISAL NAMAMGWYRQAPGERRVMVAAVSERGNAMYRESVQGRFTV TRDFTNKMVSLQMDNLKPEDTAVYYCHVLEDRVDSFHDYWG QGTQVTVSS |
| 10 | Flag tag-E1.GS.NbALFA | DYKDDDDKTRRRRHLQEIPGLSSNVATSFTWGRDSSKTSELLS GMGVSALEKEEPDSENIPQELLSNPGHPESPPRKRLKSKGSDKD FVIVRRPKLNRENFPGVSWDSLPDELLLRIFSRLRLPELLKVSGV SKRWYRLASDESLWKSLDLTGKNLHPDVTGRLLSQGVVAFRC PRSFMDQPLAGGSEVQLQESGGGLVQPGGSLRLSCTASGVTISA LNAMAMGWYRQAPGERRVMVAAVSERGNAMYRESVQGRFT VTRDFTNKMVSLQMDNLKPEDTAVYYCHVLEDRVDSFHDYW GQGTQVTVSS |
| 11 | Flag tag-E2.GS.NbALFA | DYKDDDDKTRRRRHLQEIPGLSSNVATSFTWGRDSSKTSELLS GMGVSALEKEEPDSENIPQELLSNPGHPESPPRKRLKSKGSDKD FVIVRRPKLNRENFPGVSWDSLPDELLLRIFSRLRLPELLKVSGV SKRWCRLASDESLWKTLDLTGKNLHPDVTGRLLSQGVAAFRC PRSFMDQPLAGGSEVQLQESGGGLVQPGGSLRLSCTASGVTISA LNAMAMGWYRQAPGERRVMVAAVSERGNAMYRESVQGRFT VTRDFTNKMVSLQMDNLKPEDTAVYYCHVLEDRVDSFHDYW GQGTQVTVSS |
| 12 | Flag tag-E4.GS.NbALFA | DYKDDDDKTRHRKHLQEIPDPSSNVATSFTWGWDSSKTSELPS GMGVSALEKEEPDSENIPQELLSNLGHPESPPRKRLKSKGSDKG FVIVRRPKLNRENFPGVSWVSLPDELLLGIFSCPCLPELLKVSGV CKRWCRLASDEPLWQTLDLTGKNLRPDVTGRLRSQGVIAFRCP RSFMDQPLAEGSEVQLQESGGGLVQPGGSLRLSCTASGVTISAL NAMAMGWYRQAPGERRVMVAAVSERGNAMYRESVQGRFTV TRDFTNKMVSLQMDNLKPEDTAVYYCHVLEDRVDSFHDYWG QGTQVTVSS |
| 13 | Flag tag-E5.GS.NbALFA | DYKDDDDKTRHRKHLQEIPDPSSNVATSFTWGWDSSKTSELPS GMGVSALEKKEPDSENIPQELLSNLGHPESPPRKRLKSKGSDKG FVIVRRPKLNRENFPGVSWVSLPDELLLGIFSCPCLPELLKVSGV CKRWCRLASDEPLRQTLDLTGKNLRPDVTGRLLSQGVIAFRCP RSFMDQPLAEGSEVQLQESGGGLVQPGGSLRLSCTASGVTISAL NAMAMGWYRQAPGERRVMVAAVSERGNAMYRESVQGRFTV TRDFTNKMVSLQMDNLKPEDTAVYYCHVLEDRVDSFHDYWG QGTQVTVSS |
| 14 | Flag tag-E6.GS.NbALFA | DYKDDDDKTRHRKHLQEIPDPSSNVATGFTWGWDSSKASELPS GMGVSALEKEEPDSENIPQELLSNLGHPESPPRKRLKSKGSDKG FVIVRRPKLNRENFPGVSWVSLPNELLLGIFSCRCLPELLKVSGV CKRWCRQASEEPLWQTLDLTGKNLRPDVTGRLQSQGVIAFRCP RSFMDQPLAEGSEVQLQESGGGLVQPGGSLRLSCTASGVTISAL NAMAMGWYRQAPGERRVMVAAVSERGNAMYRESVQGRFTV TRDFTNKMVSLQMDNLKPEDTAVYYCHVLEDRVDSFHDYWG QGTQVTVSS |

In addition, an expression vector was constructed in which the anti-STAT3 nanobody 'ETI01_A4' was linked to SKP2₂₋₁₇₆ and variants thereof via a 'GS' linker using the same method as above. Specifically, after treating the pCMV6 vector with a restriction enzyme, a polynucleotide encoding 'SKP2₂₋₁₇₆.GS.ETI01_A4' (SEQ ID NO: 17) and 'SKP2₂₋₁₇₆ variants (E1, E2, E4, E5).GS.ETI01_A4' (SEQ ID NO: 18 to 21) including a Flag tag in the direction from the N-terminus to the C-terminus was inserted, respectively.

**[Table 3]**

| SEQ ID NO: | Name | Amino acid sequence |
|---|---|---|
| 17 | Flag tag-SKP2(2-176).GS.ETI01_A4 | DYKDDDDKTRHRKHLQEIPDLSSNVATSFTWGWDSSKTSELL SGMGVSALEKEEPDSENIPQELLSNLGHPESPPRKRLKSKGSDK DFVIVRRPKLNRENFPGVSWDSLPDELLLGIFSCLCLPELLKVS GVCKRWYRLASDESLWQTLDLTGKNLHPDVTGRLLSQGVIAF RCPRSFMDQPLAEGSHVQLVESGGGSVQAGGSLRLSCAASGF NSGDSCIGWFRQVPGKEREGVSVISSGGEIRYYADSVKGRFTIS QDNTKNTLYLQMNSLKPEDTAMYYCATSVFLCYPRTWFNRY MYNSWGQGTQVTVSS |
| 18 | Flag tag-E1.GS.ETI01_A4 | DYKDDDDKTRRRRHLQEIPGLSSNVATSFTWGRDSSKTSELLS GMGVSALEKEEPDSENIPQELLSNPGHPESPPRKRLKSKGSDK DFVIVRRPKLNRENFPGVSWDSLPDELLLRIFSRLRLPELLKVS GVSKRWYRLASDESLWKSLDLTGKNLHPDVTGRLLSQGVVA FRCPRSFMDQPLAGGSHVQLVESGGGSVQAGGSLRLSCAASG FNSGDSCIGWFRQVPGKEREGVSVISSGGEIRYYADSVKGRFTI SQDNTKNTLYLQMNSLKPEDTAMYYCATSVFLCYPRTWFNR YMYNSWGQGTQVTVSS |
| 19 | Flag tag-E2.GS.ETI01_A4 | DYKDDDDKTRRRRHLQEIPGLSSNVATSFTWGRDSSKTSELLS GMGVSALEKEEPDSENIPQELLSNPGHPESPPRKRLKSKGSDK DFVIVRRPKLNRENFPGVSWDSLPDELLLRIFSRLRLPELLKVS GVSKRWCRLASDESLWKTLDLTGKNLHPDVTGRLLSQGVAA FRCPRSFMDQPLAGGSHVQLVESGGGSVQAGGSLRLSCAASG FNSGDSCIGWFRQVPGKEREGVSVISSGGEIRYYADSVKGRFTI SQDNTKNTLYLQMNSLKPEDTAMYYCATSVFLCYPRTWFNR YMYNSWGQGTQVTVSS |
| 20 | Flag tag-E4.GS.ETI01_A4 | DYKDDDDKTRHRKHLQEIPDPSSNVATSFTWGWDSSKTSELPS GMGVSALEKEEPDSENIPQELLSNLGHPESPPRKRLKSKGSDK GFVIVRRPKLNRENFPGVSWVSLPDELLLGIFSCPCLPELLKVS |
| | | GVCKRWCRLASDEPLWQTLDLTGKNLRPDVTGRLRSQGVIAF RCPRSFMDQPLAEGSHVQLVESGGGSVQAGGSLRLSCAASGF NSGDSCIGWFRQVPGKEREGVSVISSGGEIRYYADSVKGRFTIS QDNTKNTLYLQMNSLKPEDTAMYYCATSVFLCYPRTWFNRY MYNSWGQGTQVTVSS |
| 21 | Flag tag-E5.GS.ETI01_A4 | DYKDDDDKTRHRKHLQEIPDPSSNVATSFTWGWDSSKTSELPS GMGVSALEKKEPDSENIPQELLSNLGHPESPPRKRLKSKGSDK GFVIVRRPKLNRENFPGVSWVSLPDELLLGIFSCPCLPELLKVS GVCKRWCRLASDEPLRQTLDLTGKNLRPDVTGRLLSQGVIAF RCPRSFMDQPLAEGSHVQLVESGGGSVQAGGSLRLSCAASGF NSGDSCIGWFRQVPGKEREGVSVISSGGEIRYYADSVKGRFTIS QDNTKNTLYLQMNSLKPEDTAMYYCATSVFLCYPRTWFNRY MYNSWGQGTQVTVSS |

### Example 6. Confirmation of target protein degradation capability of fusion protein including anti-ALFA tag nanobody and SKP2 2-176 variant

The target protein degradation capability of a fusion protein including an anti-ALFA tag nanobody (NbALFA) prepared by the method of Preparation Example 2 and a SKP2₂₋₁₇₆ wild-type (WT) fragment or mutant protein was determined.

Specifically, the expression vector was transfected into human kidney cell line HEK293 cells using Lipofectamine 3000 (Invitrogen). 24 hours after transfection, the cells were washed twice with cold PBS buffer (Gibco, 10010-023), and then treated with cell lysis buffer (50 mM Tris, 10 mM EDTA, 1% SDS) containing a protease inhibitor cocktail (Thermofisher, 78440) to obtain cell extracts. The cell extracts were uniformly lysed by using a vortex and heated at 95°C for 10 minutes. The protein amount of the cell extracts was quantified by using a BCA kit (ThermoFisher, 23227), and 20 ug of protein was electrophoresed on a 4 to 12% SDS PAGE gel (Invitrogen, NW04125BOX). The separated proteins were transferred to a PVDF membrane (Invitrogen, IB24002) and blocked for 30 minutes in a blocking buffer [1×PBS-T buffer solution (1×PBS, 0.1% Tween 20) including 5% skim milk (BD, 232100). Then, the membrane was respectively treated with primary antibodies and reacted at 4°C for 16 hours. In this case, for the primary antibodies, anti-ALFA antibody (Nanotag, N1583, 1:5000), anti-α-tubulin (GeneTex, GTX628802, 1:10000) and anti-FLAG antibody (Sigma, F1804, 1:2500) were used by diluting in blocking buffer, respectively. After the primary antibody reaction, the membrane was washed three times for 5 minutes each with 1×PBS-T. Then, after treating the membrane with secondary antibodies, respectively, the membrane was reacted at room temperature for 1 hour. In this case, the secondary antibodies were anti-rabbit antibodies (CST, 7074) or anti-mouse antibodies (CST, 7076) conjugated with horseradish peroxidase (HRP) diluted in blocking buffer. After the secondary antibody reaction, the membrane was washed three times for 5 minutes each with 1×PBS-T. The expression level of each protein was determined by using an Imager (Invitrogen, iBright, CL1500) after treating with ECL (Cytiva, RPN2232 or Thermofisher, 34096) solution.

As a result, as shown in FIG. 5, it was confirmed that the expression of ALFA-Myc tag.STAT3 protein was decreased in the cell group expressing the fusion protein including SKP2₂₋₁₇₆ WT or variants thereof compared to the control group (mock). In particular, the fusion protein including E4, E5 or E6 among the SKP2₂₋₁₇₆ variants showed superior target degradation capability compared to the WT. The above experiment was repeated three times, and the results were statistically analyzed by using One-way ANOVA analysis, and it was confirmed that the fusion protein including the E4 mutant showed a statistically significant increase of about 30% in STAT3 degradation capability compared to the WT.

### Example 7. Confirmation of STAT3 protein degradation capability of fusion protein including anti-STAT3 nanobody and SKP2 2-176 or variants thereof

The target protein degradation capability of the fusion protein including the anti-STAT3 nanobody (ETI01_A4) and the SKP2₂₋₁₇₆ wild-type (WT) fragment or variants thereof, which were prepared by the method of Preparation Example 2 above, was determined. The target protein degradation capability was performed by the same method as in Example 6. In this case, an anti-c-Myc (tag) antibody was additionally used as the primary antibody.

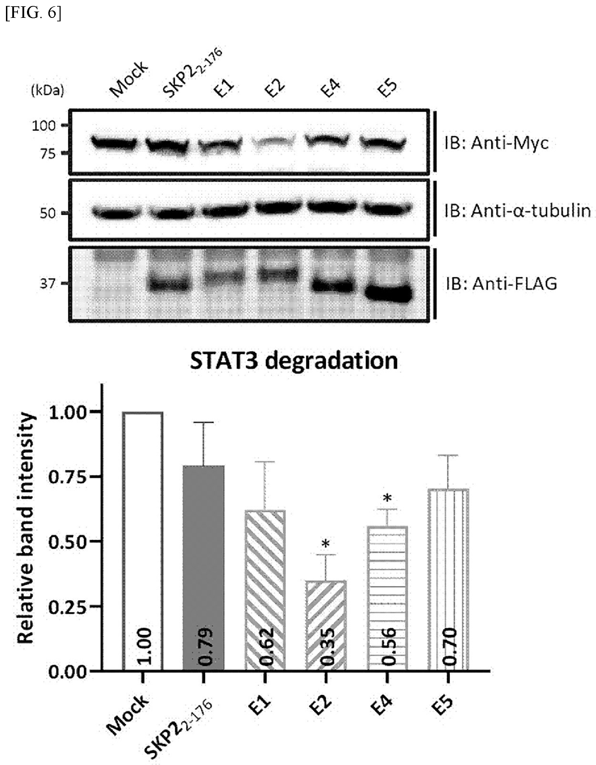

As a result, as shown in FIG. 6, it was confirmed that the expression of STAT3 protein was decreased in the cell group expressing the fusion protein including SKP2₂₋₁₇₆ WT or variants thereof compared to the control group (mock). In particular, the degradation of STAT3 protein was further increased in the cell group expressing the fusion protein including SKP2₂₋₁₇₆ variants compared to WT. The above experiment was repeated three times in total, and the results were statistically analyzed by using One-way ANOVA analysis, and it was confirmed that the fusion protein including E2 or E4 variants statistically significantly increased the STAT3 degradation capability by about 50 to 70% compared to WT.

Through the above results, it was confirmed that STAT3 was degraded more efficiently by the SKP2 variant than by the wild-type SKP2 fragment, thereby confirming that the SKP2 variant binds to SKP1 more strongly than the wild-type SKP2 fragment.

## Claims

1. An SKP2 fragment or a variant thereof.

2. The SKP2 fragment or variant thereof according to claim 1, wherein the SKP2 fragment or variant thereof comprises a binding site with SKP1.

3. The SKP2 fragment or variant thereof according to claim 1, wherein the SKP2 fragment comprises an amino acid sequence of SEQ ID NO: 1.

4. The SKP2 fragment or variant thereof according to claim 1, wherein in the SKP2 fragment variant, 7 to 14 amino acids are substituted in the SKP2 fragment.

5. The SKP2 fragment or variant thereof according to claim 1, wherein in the variant, any one amino acid selected from the group consisting of 2nd, 4th, 11th, 12th, 19th, 24th, 29th, 33rd, 44th, 59th, 78th, 98th, 102nd, 107th, 111th, 112th, 113th, 124th, 128th, 130th, 133rd, 135th, 137th, 138th, 139th, 148th, 156th, 161st, 176th amino acids and a combination thereof is substituted in an amino acid sequence of SEQ ID NO: 1.

6. The SKP2 fragment or variant thereof according to claim 5, wherein in the variant, any one amino acid selected from the group consisting of H2, K4, D11, L12, S19, W24, T29, L33, E44, L59, D78, D98, D102, G107, C111, L112, C113, C124, Y128, L130, D133, S135, W137, Q138, T139, H148, L156, I161, E176 and a combination thereof is substituted in an amino acid sequence of SEQ ID NO: 1.

7. The SKP2 fragment or variant thereof according to claim 6, wherein the variant is substituted with any one amino acid selected from the group consisting of H2R, K4R, D11G, L12P, S19G, W24R, T29A, L33P, E44K, L59P, D78G, D98V, D102N, G107R, C111R, L112P, L112R, C113R, C124S, Y128C, L130Q, D133E, S135P, W137R, Q138K, T139S, H148R, L156Q, L156R, I161A, I161V, E176G and a combination thereof in an amino acid sequence of SEQ ID NO: 1.

8. The SKP2 fragment or variant thereof according to claim 7, wherein the variant comprises any one amino acid sequence selected from the group consisting of SEQ ID NOs: 2 to 8.

9. The SKP2 fragment or variant thereof according to claim 1, wherein the variant has a binding ability with SKP1 improved by 10 to 650 times compared to an SKP2 fragment.

10. A fusion protein, comprising an SKP2 fragment or a variant thereof; and a target protein binding site.

11. The fusion protein according to claim 10, wherein the SKP2 fragment or variant thereof; and the target protein binding site are linked via a linker.

12. The fusion protein according to claim 11, wherein the fusion protein consists of Structural Formula (I) or (II) below:
N'-TB-(L)n-SR-C' (I)
N'-SR-(L)n-TB-C' (II)
wherein in Structural Formulas (I) and (II),
the N' is an N-terminus of the fusion protein,
the C' is a C-terminus of the fusion protein,
the TB is a target protein binding site (target binder),
the SR is an SKP2 fragment or a variant thereof,
the L is a peptide linker, and
the n is 0 or 1.

13. A polynucleotide, encoding the SKP2 fragment or variant thereof according to claim 1.

14. A polynucleotide, encoding the fusion protein according to claim 10.

15. A vector, loaded with the polynucleotide according to claim 13.

16. A vector, loaded with the polynucleotide according to claim 14.

17. A host cell, transformed with the vector according to claim 15 or 16.

18. A pharmaceutical composition for preventing or treating a disease, comprising a fusion protein comprising an SKP2 fragment or a variant thereof and a target protein binding site as an active ingredient.

19. A pharmaceutical composition for preventing or treating a disease, comprising the polynucleotide according to claim 14 or the vector according to claim 16 as an active ingredient.

20. The pharmaceutical composition according to claim 18 or 19, wherein the disease is selected from the group consisting of cancer, stroke, ischemic disease, peripheral vascular disease, alcoholic liver disease, hepatitis, cirrhosis, Parkinson's disease, Alzheimer's disease, fibrosis, diabetes, ALS, pathogenic disease, inflammatory disease, arthritis, anemia, genetic disorder, hyperglycemia, metabolic syndrome, lipodystrophy syndrome, dyslipidemia, insulin resistance, leptin resistance, atherosclerosis, vascular disease, hypercholesterolemia, hypertriglyceridemia, nonalcoholic fatty liver disease and obesity.

21. Use of the fusion protein according to claim 10, the polynucleotide according to claim 14 or the vector according to claim 16 in the prevention or treatment of a disease.

22. A method for preventing or treating a disease, comprising administering the fusion protein according to claim 10, the polynucleotide according to claim 14 or the vector according to claim 16 to a subject.
